# EUROPÄISCHE PATENTANMELDUNG

(11) **EP 0 682 019 A1**
(43) Veröffentlichungstag der Anmeldung: **15.11.1995**
(21) Anmeldenummer: 95106242.1
(22) Anmeldetag: 26.04.1995
(51) Int. Cl.: C07D 251/44, B01J 19/24, C09B 62/04, C09B 62/085

(54) **Verfahren zur kontinuierlichen Umsetzung von Cyanurfluorid mit Aminen**

(30) Priorität: 06.05.1994 DE 4416017
(71) Anmelder: HOECHST AKTIENGESELLSCHAFT, D-65929 Frankfurt am Main (DE)
(72) Erfinder: Angenendt, Heinrich, Dr., D-65510 Idstein (DE); Fuchs, Hermann, Dr., D-61462 Königstein (DE); Grötsch, Georg, Dr., D-65812 Bad Soden (DE); Strohmeyer, Eckhard, D-65817 Eppstein (DE); Tronich, Wolfgang, Dr., D-65817 Eppstein (DE)

(57) **Zusammenfassung**

Die Erfindung betrifft ein Verfahren zur Herstellung von 1-Amino-3,5-difluor-s-triazinylverbindungen aus Cyanurfluorid und Aminen, dadurch gekennzeichnet, daß Cyanurfluorid und eine wäßrige Aminlösung gleichzeitig und kontinuierlich in konstanten Massenströmen, die ein Molverhältnis von Cyanurfluorid zu Amin zwischen 1:1 und 1,5:1 gewährleisten, in eine Rohrleitung mit eingebauten statischen Mischelementen eingeleitet werden, wobei eine intensive Zwangsvermischung stattfindet und daß der pH-Wert des wäßrigen Reaktionsgemisches nach Verlassen des statischen Mischers mit anorganischen alkalisch wirkenden Mitteln auf 5,5 bis 7,0 eingestellt wird.

## Beschreibung

Die Erfindung betrifft ein Verfahren zur kontinuierlichen Umsetzung von Cyanurfluorid mit Aminen, wobei Cyanurfluorid und eine wäßrige Aminlösung in einen statischen Mischer eingeleitet werden und in der Reaktionsmischung nach Verlassen der Mischzone eine pH-Wert Regulierung entweder in einem weiteren statischen Mischer oder in einem Rührkessel vorgenommen wird.

Aus der DE-C2 2 746 109 ist ein Verfahren zur kontinuierlichen Umsetzung von Cyanurfluorid mit Aminobenzol- und Aminonaphthalin-sulfonsäuren bekannt, wobei als Reaktor ein kontinuierlich durchflossener Rührkessel mit vollständiger Rückvermischung der Reaktionsmasse im Reaktor verwendet wird. Mit der dort beschriebenen Arbeitsweise werden jedoch aufgrund erheblicher Nebenproduktebildung nur beschränkte Reaktionsselektivitäten erzielt.

Die EP-A2 0172 790 beschreibt ein Verfahren zur kontinuierlichen Umsetzung von Cyanurfluorid mit Aminen, in dem Cyanurfluorid und wäßrige Lösungen der Amine gleichzeitig in einen ersten Reaktor geleitet werden, in welchem eine intensive Durchmischung stattfindet, und die Reaktionsmischung anschließend in einen zweiten Reaktor geleitet wird, in welchem nur geringe Rückvermischung aber gute radiale Vermischung eintritt, und dort die Umsetzung zu Ende geführt wird, wobei der Umsatz in dem ersten Reaktor maximal 50 % betragen darf.

Diese Arbeitsweise ist dadurch nachteilig, daß ein zweiter Reaktor benötigt wird, um die Reaktion zu Ende zu führen und daß der Anteil der entstehenden Nebenprodukte gegenüber dem in der DE-C2 2 746 109 beschriebenen Verfahren zwar vermindert, aber noch immer unbefriedigend hoch ist. Ein gegenüber dem vorgenannten Stand der Technik verbessertes Verfahren ist mit der DE-C2 4 016 159 bekanntgeworden. Danach wird bei der kontinuierlichen Umsetzung von Cyanurfluorid mit Aminen in wäßriger Lösung in einer einzigen Reaktionsstufe eine Umsetzung von 95 % erreicht, wenn man die Edukte gleichzeitig mit unterschiedlichen Geschwindigkeiten in einen Reaktor so einleitet, daß durch die Differenz der Einströmgeschwindigkeiten eine intensive Vermischung bewirkt und die Reaktion im wesentlichen in diesem Reaktor abgeschlossen wird. Der Unterschied der Einströmgeschwindigkeiten ist dadurch beschrieben, daß das Cyanurfluorid mit einer Reynolds-Zahl von mindestens 10000, die wäßrige Aminlösung mit einer solchen von mindestens 2500 in den Reaktor einströmen und das Verhältnis der Reynoldszahlen des Cyanurfluoridstromes und des Aminlösungsstromes 2,5:1 bis 10:1 vorzugsweise 4 bis 8:1 beträgt. Der Reaktor zur Durchführung des Verfahrens ist gekennzeichnet durch ein Rohr, in das zur Einführung von Cyanurfluorid eine Düse axial und eine oder mehrere Düsen oder eine Ringdüse konzentrisch dazu, durch welche die wäßrigen Aminlösungen eingeführt werden, eingebaut sind. Als weitere Bedingung für das Verfahren gilt, daß das Verhältnis des Gesamtquerschnitts des Reaktionsrohres zum Einströmquerschnitt der Cyanurfluoriddüse bei Werten von 225 bis 60000 eingehalten wird. Darüber hinaus ist in der zugehörigen Patentanmeldung EP-A1 0 458 110 angegeben, daß die Differenz der Strömungsgeschwindigkeiten zwischen Cyanurfluorid- und Aminlösungsstrom mindestens 20 m/s, vorzugsweise 40 m/s beträgt.
Diese apparative Anordnung wirft einige technische Probleme hinsichtlich der praktischen Durchführung auf:
Einmal erfordert die Gewährleistung einer hohen Einströmungsgeschwindigkeit für das Cyanurfluorid in den Reaktor extrem kleine Düsenaustrittsquerschnitte. Damit ist eine extreme Reinheit des zufließenden Cyanurfluorids hinsichtlich der geringsten Belastung mit unlöslichen Partikeln notwendig, die zu Verstopfungen und damit Zwangsunterbrechung des kontinuierlichen Laufs führen können.

Weiterhin müssen bei einem Produktwechsel, d.h. der Umsetzung eines anderen Amins mit Cyanurfluorid, die Parameter neu optimiert werden. Da davon auszugehen ist, daß der Ringdüsenquerschnitt für die Zuführung der Aminlösungen fix ist, muß der Düsenquerschnitt für die Cyanurfluoridzuführung variabel sein; das bedeutet, es müssen Düsen mit unterschiedlichen Öffnungsquerschnitten vorgehalten werden.

Es bestand somit ein großer Bedarf nach einem Verfahren, das es erlaubt, die kontinuierliche Umsetzung von Cyanurfluorid mit Aminen zu 1-Amino-3,5-difluor-s-triazinyl-Verbindungen in vergleichbarer Ausbeute und Reinheit, in einer einfach und technisch nicht aufwendigen Weise durchzuführen.

Die Aufgabe wird gelöst durch ein Verfahren zur Herstellung von 1-Amino-3,5-difluor-s-triazinylverbindungen aus Cyanurfluorid und Aminen, dadurch gekennzeichnet, daß Cyanurfluorid und eine wäßrige Aminlösung gleichzeitig und kontinuierlich in konstanten Massenströmen, die ein Molverhältnis von Cyanurfluorid zu Amin zwischen 1:1 und 1,5:1 gewährleisten, in eine Rohrleitung mit eingebauten statischen Mischelementen eingeleitet werden, wobei eine intensive Zwangsvermischung stattfindet, und daß der pH-Wert des wäßrigen Reaktionsgemisches nach Verlassen des statischen Mischers mit anorganischen alkalisch wirkenden Mitteln auf 5,5 bis 7,0 eingestellt wird.

Dadurch, daß die Edukte gleichzeitig und kontinuierlich mit Strömungsgeschwindigkeiten, die ein nahezu konstantes molares Verhältnis vorgeben, in einen statischen Mischer mit intensiver Zwangsdispergierung der Phasen ineinander eindosiert werden, läuft die Reaktion ohne Rückvermischung auf der kürzest möglichen Reaktionsstrecke vollständig ab. Mit der Verkürzung der Reaktionsstrecke ergibt sich als ein erfindungsgemäßer Vorteil die einfache technische Möglichkeit der Unterdrückung der qualitätsmindernden Folgereaktion, nämlich der protonenkatalysierten hydrolytischen Austauschreaktion von Fluoratomen gegen Hydroxylgruppen an den Difluortriazinylaminoverbindungen. Dies wird dadurch erreicht, indem innerhalb von 5 s, insbesondere innerhalb von 2 s, bevorzugt innerhalb 1 s nach Verlassen der Reaktionsstrecke eine pH-Wert Einstellung auf 5,5 bis 7,0 durchgeführt wird, wobei man mit wäßrigen Alkalien kontinuierlich in einem nachgeschalteten Rührkessel neutralisiert. Eine weitere Möglichkeit besteht darin, die pH-Wert-Einstellung auf 5,5 bis 7,0 nach dem Austritt des Reaktionsgemisches aus dem statischen Mischer durch gleichzeitiges und kontinuierliches Zuführen des Reaktionsgemisches und von wäßrigen Lösungen von anorganischen alkalisch wirkenden Mitteln in einen zweiten statischen Mischer durchzuführen, und die zuzuführende Menge an Alkali mittels pH-Wertbestimmung im Reaktionsgemisch nach Austritt aus dem zweiten statischen Mischer zu regeln. Die Verweilzeit im ersten statischen Mischer beträgt 0,01 bis 1 s, insbesondere 0,05 bis 0,5 s, die Verweilzeit im zweiten statischen Mischer für die pH-Wert-Einstellung ist analog. Die Reaktion mit dem Cyanurfluorid wie auch die anschließende pH-Wert-Einstellung werden bei 0 bis 20°C, insbesondere bei 0 bis 5°C durchgeführt.

Es ist vorteilhaft, wenn die statischen Mischelemente zum Dispergieren von ineinander unlöslichen Flüssigkeiten im turbulenten Strömungsbereich ausgelegt sind.

Es hat sich hierbei als günstig erwiesen, statische Mischelemente zu verwenden, die aus geriffelten Lamellen aufgebaut sind, die offene, sich kreuzende Kanäle bilden.

In vielen Fällen hat es sich bewährt, wenn die Länge der hintereinander geschalteten Mischelemente, die die Mischstrecke bilden, das 2- bis 12-fache ihres Durchmessers beträgt.

Besonders vorteilhaft ist es, die beiden Eduktströme Cyanurfluorid und wäßrige Aminlösung in geringem Abstand vor den statischen Mischelementen zusammenzuführen. Da sich die zu vermischenden Volumen in weiten Bereichen unterscheiden, ist es weiterhin vorteilhaft, das wesentlich kleinere Cyanurfluoridvolumen durch eine enge Zuleitung, weitgehend zentriert und axial eingebaut, in die Zuleitung für das wesentlich größe Volumen der wäßrigen Aminlösungen, in der gleichen Strömungsrichtung zuzuführen. Als statische Mischelemente sind alle mechanischen Einbauten in einer Rohrleitung geeignet, die nach möglichst kürzester Durchflußstrecke ohne nennenswerten Druckverlust eine bestmögliche disperse Verteilung des flüssigen Cyanurfluorids für den optimalen Umsatz mit dem im Wasser gelösten Amin ergeben.

Nach dem erfindungsgemäßen Verfahren lassen sich Aminogruppen enthaltende, durch die weitere Substitution mit einer oder mehreren Sulfogruppen wasserlösliche chromophore Systeme mit Cyanurfluorid in sehr guten Ausbeuten im molaren Verhältnis 1:1 umsetzen.

Besonders geeignet ist das erfindungsgemäße Verfahren für die Umsetzung von Cyanurfluorid mit wasserlöslichen aminogruppenhaltigen chromophoren Systemen der folgenden allgemeinen Formeln (I) bis (III): 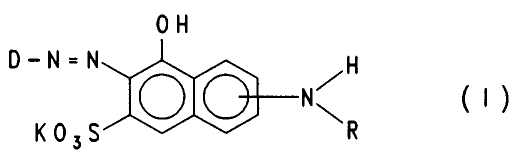
Darin bedeutet D einen Phenyl- oder Naphthylrest mit einer oder mehreren Sulfonsäuregruppen sowie Alkyl- oder Alkoxysubstituenten, K steht für die Kationen Li⁺, Na⁺, K⁺ und R bedeutet ein Wasserstoffatom oder den Methyl- oder Ethylrest. 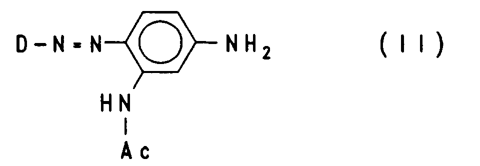
Darin entspricht D den für die allgemeine Formel (1) angegebenen Resten, Ac bedeutet einen Acylrest wie beispielsweise dem Amidocarbonyl-, Acetyl- oder Propionylrest. 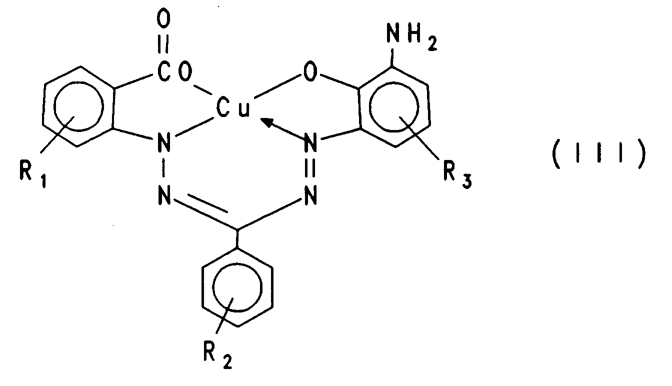
Darin bedeuten R₁, R₂ und R₃ Wasserstoffsubstituenten, Alkyl- oder Alkoxyreste wie Methyl- oder Methoxygruppen sowie Sulfonsäuregruppen, wobei in Summe pro Molekül mindestens 2 Sulfonsäuregruppen vorhanden sind.

Die wasserlöslichen Aminogruppen enthaltenden chromophoren Systeme werden als wäßrige Lösungen eingesetzt. Man kann das Cyanurfluorid im Überschuß einsetzen. Zweckmäßig ist es, Cyanurfluorid und Amin im Molverhältnis 1:1 bis 1,5:1, vorzugsweise 1:1 bis 1,2:1, insbesondere 1,02:1 bis 1,15:1, bevorzugt 1,05:1 bis 1,10:1 einzusetzen. Der Aminlösung kann eine Puffersubstanz zugesetzt werden, welche während der Umsetzung mit dem Cyanurfluorid, in Abhängigkeit der gewählten Substanz, einen pH-Wert zwischen 1 und 8, vorzugsweise zwischen 3 und 5 einstellt. Geeignete Puffersubstanzen sind Alkalifluoride, -borate und -phosphate. In einer besonderen erfindungsgemäßen Ausführungsform des Verfahrens wird jedoch vorteilhaft auf die Puffersubstanzen verzichtet und statt dessen der pH-Wert des Reaktionsgemisches innerhalb von max. 5 s, vorzugsweise innerhalb von 2 s, nach verlassen des statischen Mischers entweder in einem Rührkessel oder in einem zweiten statischen Mischer auf 5,5 bis 7,0 vorzugsweise auf 6,0 bis 6,7 eingestellt mit wäßrigen Alkalien, beispielsweise verdünnter wäßriger Natronlauge oder wäßriger Sodalösung.

Die Reaktionsprodukte aus 1 Mol aminogruppenhaltigem Chromophor und 1 Mol Cyanurfluorid werden vorzugsweise ohne Zwischenisolierung weiterverarbeitet, z.B. zu Reaktivfarbstoffen durch die Umsetzung mit einem weiteren Amin, wobei dieses Amin gegebenenfalls Gruppierungen enthalten kann, welche mit Cellulosefasern reagieren können.

Entsprechend den verfahrenstechnischen Vorgaben des vorgenannten Standes der Technik für die Zielreaktion, war es nicht vorhersehbar, daß die angestrebte möglichst selektive, d.h. möglichst an Neben- und Folgeprodukten arme Umsetzung von Aminen in wäßriger Lösung mit Cyanurfluorid mit einfachen, kommerziell zugänglichen technischen Systemen erreicht werden kann.

### Beispiel 1

In einen statischen Mischer mit einem Querschnitt von 176 mm² und 8 hintereinander angeordneten Mischelementen mit einer Gesamtlänge des 8-fachen Durchmessers werden gleichzeitig und kontinuierlich über getrennte Zuleitungen 9,3 l/h Cyanurfluorid mit einer Temperatur von 20 ± 5°C und 500 l/h einer wäßrigen Lösung mit einer Temperatur von 0 bis 3°C, welche 0,183 Mol/l des Farbstoffes der folgenden Formel (1) enthält, eingeleitet: 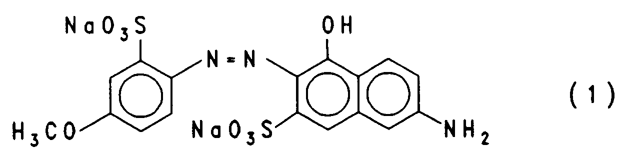
Die Reinheit des Farbstoffes der Formel I betrug gemäß HPLC-analytischer Bestimmung 95,0 Flächen-%.

Die Verweilzeit des Reaktionsgemisches in der statischen Mischerstrecke beträgt 0,15 s. Die wäßrige Reaktionsmischung, welche mit einer Temperatur von 4 bis 5°C die Mischerstrecke verläßt, enthält den Difluortriazinyl-Farbstoff der Formel (2) 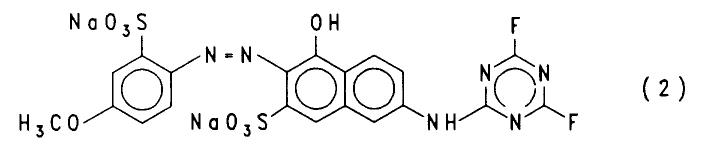
mit einem HPLC-analytisch bestimmten Anteil von 90,5 Flächen-%. Daneben werden durch protonenkatalysierte Hydrolyse bereits im statischen Mischer gebildete Folgeprodukte aus (2), der Monofluor-monohydroxytriazinylfarbstoff mit 2,3 Flächen-% und der Dihydroxytriazinylfarbstoff mit 0,5 Flächen-% per HPLC gefunden. Ein Teil des Ausgangsfarbstoffes (1) mit 3,5 Flächen-% bleibt nicht umgesetzt. Damit ergibt sich ein Umsatz des Farbstoffes (1) mit > 93 %, gerechnet über die Flächen-% nach HPLC im statischen Mischer.

Die Reaktionsmischung wird innerhalb von 1,2 s in einen Rührkessel geleitet, in dem kontinuierlich, während des Einlaufs, der pH-Wert mit einer wäßrigen Sodalösung auf 6,0 bis 6,7 eingestellt wird.
Anstatt einen Rührkessel zu benutzen, ist es auch möglich, den Strom der Reaktionsmischung nach Verlassen der statischen Mischerstrecke gleichzeitig und kontinuierlich mit einer wäßrigen Sodalösung über ein Einleitrohr zu versetzen und über eine zweite Mischerstrecke zu führen, wobei die für die pH-Stellung auf 6,0 bis 6,7 erforderliche Zulaufmenge an Sodalösung anhand einer pH-Messung in der Reaktionsmischung nach Verlassen des zweiten statischen Mischers geregelt wird.
Durch diese Maßnahmen wird die hydrolytische Folgereaktion ausgehend von Farbstoff (2) zurückgedrängt.
Die weitere Umsetzung des Reaktionsgemisches kann mit Aminen erfolgen. Z.B. ergibt die Reaktion mit der aliphatischen Aminoverbindung 3-Aminopropyl-β-sulfatoethylsulfon bei 0 bis 10°C und einem pH-Wert zwischen 7,5 und 8,0 folgenden Farbstoff (3), 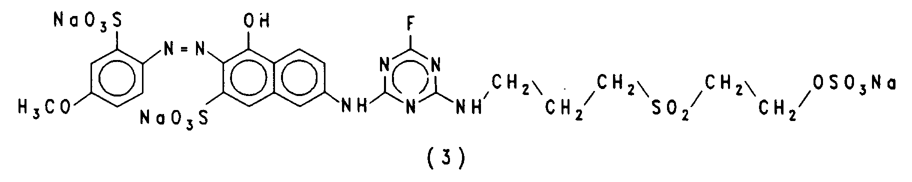
welcher aufgrund des pH-Bereichs der Umsetzung durch Abspaltung von Natriumsulfat aus dem β-Sulfatoethylsulfon-Rest einen Anteil des entsprechenden Vinylsulfon-Farbstoffes enthält. Beide Produkte sind hinsichtlich der Reaktivität mit Cellulosefasern gleichwertig. Damit erhält man einen Reaktivfarbstoff, der aufgrund zweier faserreaktiven Zentren, der unter alkalischen Färbebedingungen aus dem β-Sulfatoethylsulfonyl-Rest gebildeten Vinylsulfongruppe sowie dem Monofluortriazinrest, ausgezeichnete färberische Ausbeuten und hohe Gebrauchsechtheiten der Färbungen auf Cellulosefasern ergibt.

### Beispiel 2

In einen statischen Mischer mit einem Querschnitt von 176 mm² und 8 hintereinander angeordneten Mischelementen mit einer Gesamtlänge des 8-fachen Durchmessers werden gleichzeitig und kontinuierlich über getrennte Zuleitungen 6,8 l/h Cyanurfluorid mit einer Temperatur von 20 ± 5°C und 500 l/h einer wäßrigen Lösung mit einer Temperatur von 0 bis 3°C, welche 0,134 Mol/l des Farbstoffes der folgenden Formel (4) enthält, eingeleitet: 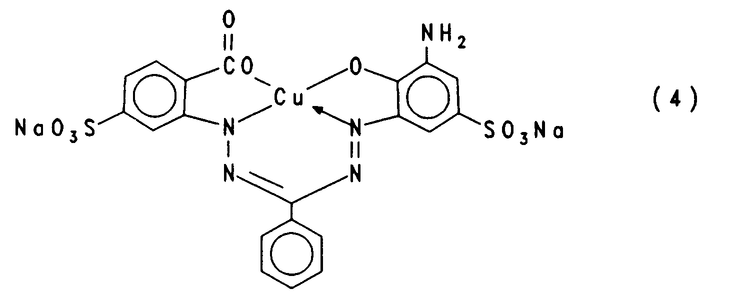
Die Reinheit des Farbstoffes der Formel (4) beträgt gemäß HPLC-analytischer Bestimmung 82,3 Flächen-%.

Die Verweilzeit des Reaktionsgemisches in der statischen Mischerstrecke beträgt 0,15 s. Die wäßrige Reaktionsmischung, welche mit einer Temperatur von 4 bis 5°C die Mischerstrecke verläßt, enthält den Difluortriazinyl-Farbstoff der Formel (5) 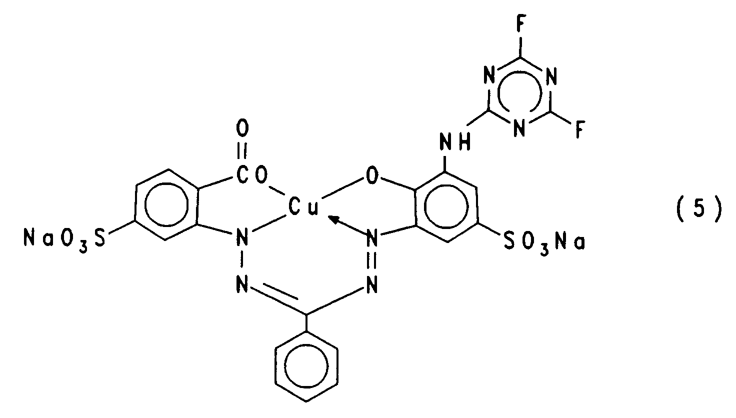
mit einem HPLC-analytisch bestimmten Anteil von 72,6 Flächen-%. Daneben werden durch protonenkatalysierte Hydrolyse bereits im statischen Mischer gebildete Folgeprodukte aus (5) der Monofluor-monohydroxytriazinylfarbstoff mit 2,7 Flächen-% und der Dihydroxytriazinylfarbstoff mit ca. 0,2 Flächen-% per HPLC gefunden.

Damit ergibt sich ein Umsatz des Farbstoffes (4) mit > 90 %, gerechnet über die Flächen-% nach HPLC im statischen Mischer. Die Reaktionsmischung wird innerhalb von 1,2 s in einen Rührkessel geleitet, in dem kontinuierlich, während des Einlaufs, der pH-Wert mit einer wäßrigen Sodalösung auf 6,0 bis 6,7 eingestellt wird.

Die weitere Umsetzung des Reaktionsgemisches kann wie in Beispiel 1 beschrieben mit der aliphatischen Aminoverbindung 3-Aminopropyl-β-sulfatoethylsulfon bei 0 bis 10°C und einem pH-Wert zwischen 7,5 und 8,0 zu folgendem Farbstoff (6) erfolgen, 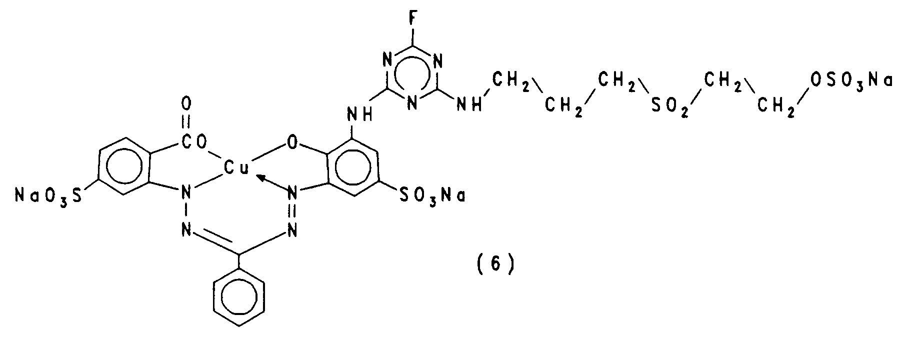
welcher aufgrund des pH-Bereichs der Umsetzung durch Abspaltung von Natriumsulfat aus dem β-Sulfatoethylsulfon-Rest einen Anteil des entsprechenden Vinylsulfon-Farbstoffes enthält. Beide Produkte sind hinsichtlich der Reaktivität mit Cellulosefasern gleichwertig. Damit erhält man einen Reaktivfarbstoff, der aufgrund zweier faserreaktiven Zentren, der unter alkalischen Färbebedingungen aus dem β-Sulfatoethylsulfonyl-Rest gebildeten Vinylsulfongruppe sowie dem Monofluortriazinrest, ausgezeichnete färberische Ausbeuten und hohe Gebrauchsechtheiten der Färbungen auf Cellulosefasern ergibt.

### Beispiel 3

In einen statischen Mischer mit einem Querschnitt von 176 mm² und 8 hintereinander angeordenten Mischelementen mit einer Gesamtlänge des 8-fachen Durchmessers werden gleichzeitig und kontinuierlich über getrennte Zuleitungen 7,4 l/h Cyanurfluorid mit einer Temperatur von 20 ± 5°C und 500 l/h einer wäßrigen Lösung mit einer Temperatur von 0 bis 3°C, welche 0,145 Mol/l des Farbstoffes der folgenden Formel (7) enthält, eingeleitet: 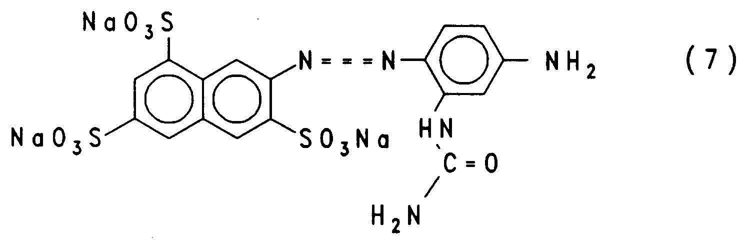
Die Reinheit des Farbstoffes der Formel (7) beträgt gemäß HPLC-analytischer Bestimmung 80,7 Flächen-%.
Die Verweilzeit des Reaktionsgemisches in der statischen Mischerstrecke beträgt 0,15 s. Die wäßrige Reaktionsmischung, welche mit einer Temperatur von 4 bis 5°C die Mischerstrecke verläßt, enthält den Difluortriazinyl-Farbstoff der Formel (8) 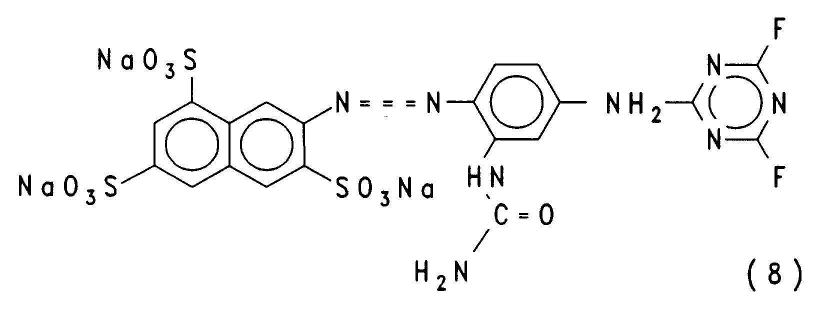
mit einem HPLC-analytisch bestimmten Anteil von 74 Flächen-%. Daneben werden durch protonenkatalysierte Hydrolyse bereits im statischen Mischer gebildete Folgeprodukte aus (8), der Monofluor-monohydroxytriazinylfarbstoff mit 2,5 Flächen-% und der Dihydroxytriazinylfarbstoff mit ca. 0,2 Flächen-% per HPLC gefunden.

Damit ergibt sich ein Umsatz des Farbstoffes (7) mit > 90 %, gerechnet über die Flächen-% nachHPLC im statischen Mischer. Die Reaktionsmischung wird innerhalb von 1,2 s in einen Rührkessel geleitet, in dem kontinuierlich, während des Einlaufs, der pH-Wert mit einer wäßrigen Sodalösung auf 6,0 bis 6,7 eingestellt wird.

Die weitere Umsetzung des Reaktionsgemisches kann wie in Beispiel 1 beschrieben mit der aliphatischen Aminoverbindung 3-Aminopropyl-β-sulfatoethylsulfon bei 0 bis 10°C und einem pH-Wert zwischen 7,5 und 8,0 zu folgendem Farbstoff (9) erfolgen, 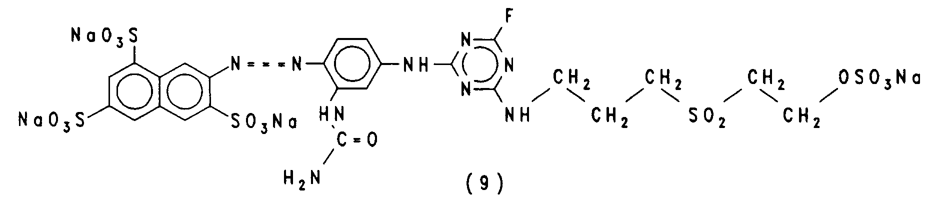
welcher aufgrund des pH-Bereichs der Umsetzung durch Abspaltung von Natriumsulfat aus dem β-Sulfatoethylsulfon-Rest einen Anteil des entsprechenden Vinylsulfon-Farbstoffes enthält. Beide Produkte sind hinsichtlich der Reaktivität mit Cellulosefasern gleichwertig. Damit erhält man einen Reaktivfarbstoff, der aufgrund zweier faserreaktiven Zentren, der unter alkalischen Färbebedingungen aus dem β-Sulfatoethylsulfonyl-Rest gebildeten Vinylsulfongruppe sowie dem Monofluortriazinrest, ausgezeichnete färberische Ausbeuten und hohe Gebrauchsechtheiten der Färbungen auf Cellulosefasern ergibt.

## Patentansprüche

1. Verfahren zur Herstellung von 1-Amino-3,5-difluor-s-triazinylverbindungen aus Cyanurfluorid und Aminen, dadurch gekennzeichnet, daß Cyanurfluorid und eine wäßrige Aminlösung gleichzeitig und kontinuierlich in konstanten Massenströmen, die ein Molverhältnis von Cyanurfluorid zu Amin zwischen 1:1 und 1,5:1 gewährleisten, in eine Rohrleitung mit eingebauten statischen Mischelementen eingeleitet werden, wobei eine intensive Zwangsvermischung stattfindet und daß der pH-Wert des wäßrigen Reaktionsgemisches nach Verlassen des statischen Mischers mit anorganischen alkalisch wirkenden Mitteln auf 5,5 bis 7,0 eingestellt wird.

2. Verfahren nach Anspruch 1, dadurch gekennzeichnet, daß der pH-Wert innerhalb von 5 s, insbesondere innerhalb von 2 s, bevorzugt innerhalb 1 s nach Verlassen des statischen Mischers mit anorganischen alkalisch wirkenden Mitteln in einem Rührkessel auf 5,5 bis 7,0 eingestellt wird.

3. Verfahren nach Anspruch 1, dadurch gekennzeichnet, daß die pH-Wert-Einstellung auf 5,5 bis 7,0 nach dem Austritt des Reaktionsgemisches aus dem statischen Mischer durch gleichzeitiges und kontinuierliches Zuführen des Reaktionsgemisches und von wäßrigen Lösungen von anorganischen alkalisch wirkenden Mitteln in einen zweiten statischen Mischer durchgeführt, und die zuzuführende Menge an Alkali mittels pH-Wertbestimmung im Reaktionsgemisch nach Austritt aus dem zweiten statischen Mischer geregelt wird.

4. Verfahren nach mindestens einem der Ansprüche 1 bis 3, dadurch gekennzeichnet, daß die Verweilzeit im ersten statischen Mischer 0,01 bis 1 s, insbesondere 0,05 bis 0,5 s beträgt.

5. Verfahren nach Anspruch 3, dadurch gekennzeichnet, daß die Verweilzeit im zweiten statischen Mischer 0,01 bis 1 s, insbesondere 0,05 bis 0,5 s beträgt.

6. Verfahren nach mindestens einem der Ansprüche 1 bis 5, dadurch gekennzeichnet, daß die Reaktion des Amins mit dem Cyanurfluorid und die pH-Wert-Einstellung bei Temperaturen von 0 bis 5°C durchgeführt werden.

7. Verfahren nach mindestens einem der Ansprüche 1 bis 6, dadurch gekennzeichnet, daß die statischen Mischelemente zum Dispergieren von ineinander unlöslichen Flüssigkeiten im turbulenten Strömungsbereich ausgelegt sind, insbesondere daß die statischen Mischelemente aus geriffelten Lamellen aufgebaut sind, die offene, sich kreuzende Kanäle bilden.

8. Verfahren nach mindestens einem der Ansprüche 1 bis 7, dadurch gekennzeichnet, daß die Länge der hintereinandergeschalteten Mischelemente, die die Mischstrecke bilden, das 2- bis 12-fache des Durchmessers beträgt.

9. Verfahren nach mindestens einem der Ansprüche 1 bis 8, dadurch gekennzeichnet, daß Aminogruppen enthaltende, durch die weitere Substitution mit einer oder mehreren Sulfogruppen wasserlösliche chromophore Systeme eingesetzt werden.

10. Verfahren nach mindestens einem der Ansprüche 1 bis 8, dadurch gekennzeichnet, daß als Amin ein Chromophor der allgemeinen Formel (I) 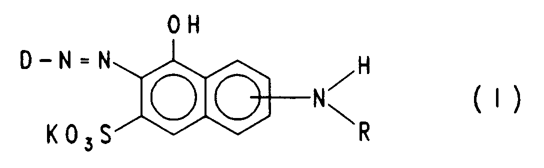 worin D Phenyl oder Naphthyl mit einer oder mehreren Sulfonsäuregruppen sowie Alkyl- oder Alkoxysubstituenten, K Li⁺, Na⁺, K⁺ und R Wasserstoff, Methyl oder Ethyl bedeutet, eingesetzt wird.

11. Verfahren nach mindestens einem der Ansprüche 1 bis 8, dadurch gekennzeichnet, daß als Amin ein Chromophor der allgemeinen Formel (II), 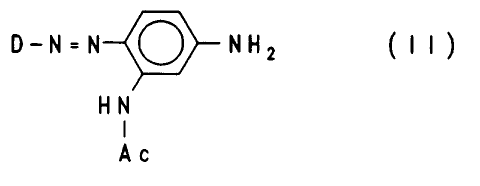 worin D Phenyl oder Naphthyl mit einer oder mehreren Sulfonsäuregruppen sowie Alkyl- oder Alkoxysubstituenten und Ac einen Acylrest, insbesondere Aminocarbonyl-, Acetyl- oder Propionylrest bedeutet, eingesetzt wird.

12. Verfahren nach mindestens einem der Ansprüche 1 bis 8, dadurch gekennzeichnet, daß als Amin ein Chromophor der allgemeinen Formel (III) 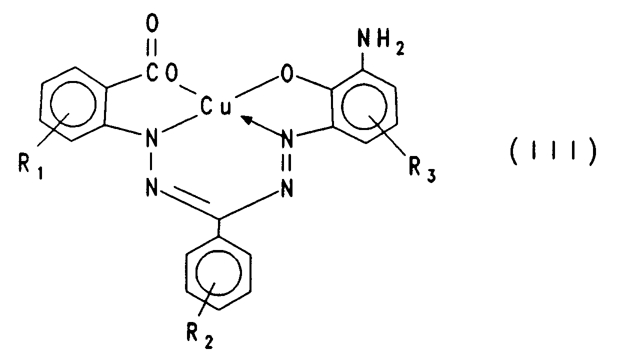 worin R₁, R₂ und R₃ Wasserstoff, Alkyl oder Alkoxy, insbesondere Methyl oder Methoxy sowie Sulfonsäuregruppen, wobei in Summe pro Molekül mindestens 2 Sulfonsäuregruppen vorhanden sind, bedeutet, eingesetzt wird.

13. Verfahren nach mindestens einem der Ansprüche 1 bis 12, dadurch gekennzeichnet, daß Cyanurfluorid und Amin im Molverhältnis 1:1 bis 1,2:1, insbesondere 1,02:1 bis 1,15:1, bevorzugt 1,05:1 bis 1,10:1 eingesetzt werden.

14. Verfahren nach mindestens einem der Ansprüche 1 bis 13, dadurch gekennzeichnet, daß der pH-Wert auf 6,0 bis 6,7 eingestellt wird.

15. Verfahren nach mindestens einem der Ansprüche 1 bis 14, dadurch gekennzeichnet, daß als anorganisch alkalisch wirkendes Mittel wäßrige Natronlauge oder Sodalösung eingesetzt wird.
